(19) Europäisches Patentamt
European Patent Office
Office européen des brevets

(11) Veröffentlichungsnummer: **0 212 138**
**A2**

(12) # EUROPÄISCHE PATENTANMELDUNG

(21) Anmeldenummer: **86108564.5**

(22) Anmeldetag: **24.06.86**

(51) Int. Cl.⁴: **C 07 D 303/44**, C 07 D 301/14

(30) Priorität: **05.08.85 DE 3528002**

(43) Veröffentlichungstag der Anmeldung: **04.03.87**
**Patentblatt 87/10**

(84) Benannte Vertragsstaaten: **AT CH DE FR GB IT LI NL SE**

(71) Anmelder: **Degussa Aktiengesellschaft,**
**Weissfrauenstrasse 9, D-6000 Frankfurt am Main 1 (DE)**

(72) Erfinder: **Boehme, Georg, Nordring 49,**
**D-6458 Rodenbach 1 (DE)**
Erfinder: **Hofen, Willi, Südring 54,**
**D-6458 Rodenbach 1 (DE)**
Erfinder: **Grund, Andreas, Dr., Rilkeweg 15,**
**D-6100 Darmstadt 1 (DE)**
Erfinder: **Petsch, Heinrich, Tulpenstrasse 23,**
**D-6450 Hanau 8 (DE)**
Erfinder: **Prescher, Günter, Dr., Liesingstrasse 2,**
**D-6450 Hanau 9 (DE)**

(54) **Verfahren zur Herstellung eines cycloaliphatischen Diepoxids.**

(57) Mit Hilfe von benzolischer Perpropionsäurelösung, die auch in ungereinigter Form mit bestimmten Maximalgehalten an Wasserstoffperoxid, Wasser und Mineralsäure verwendet werden kann, läßt sich das Diepoxid der Formel

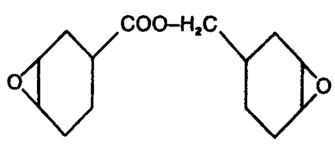

aus dem entsprechenden Diolefin in technisch einfacher Form herstellen.

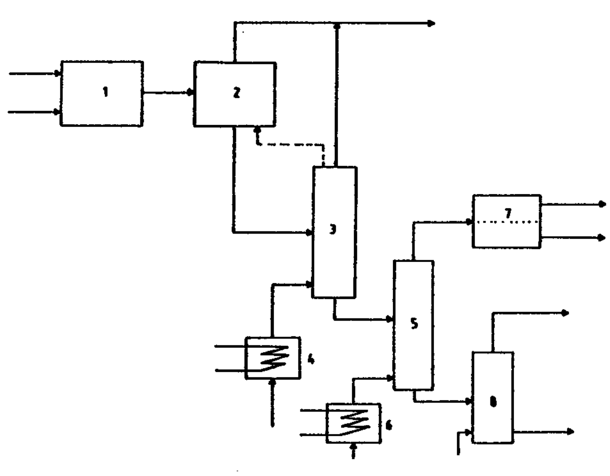


EP 0 212 138 A2

D e g u s s a Aktiengesellschaft
Weissfrauenstrasse 9, 6000 Frankfurt

Verfahren zur Herstellung eines cycloaliphatischen Diepoxids

Beschreibung:

Die vorliegende Erfindung betrifft ein Verfahren zur Herstellung eines cycloaliphatischen Diepoxids der Formel

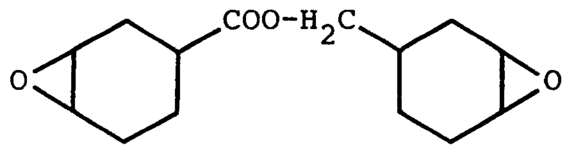

(I)

durch Epoxydation eines Diolefins der Formel

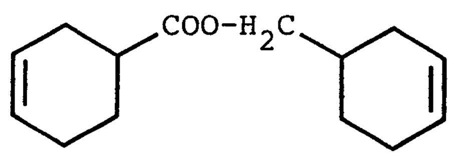

(II)

mit einer Percarbonsäure in organischem Lösungsmittel sowie zur Aufarbeitung des dabei entstehenden Reaktionsgemisches.

Das cycloaliphatische Diepoxid obenstehender Struktur findet zunehmend Verwendung als Bestandteil von Epoxidharzen,

die sich vor allem als Isolations- und Vergußmaterial auf dem Elektro- und Elektroniksektor sowie für strahlenhärtbare Lack- und Beschichtungssysteme eignen. Gerade letztere sind deshalb besonders umweltfreundlich, da sie lösungsmittelfrei sind.

Es ist seit langem bekannt, Epoxide durch Umsetzung von Olefinen mit Chlor im alkalischen Medium und nachfolgender Behandlung mit Basen herzustellen (Ullmann's Enzyklopädie der technischen Chemie, 3. Auflage, Band 10, Seite 565). Der wesentliche Nachteil dieses Verfahrens sind die erheblichen Mengen umweltbelastender Abwässer, die bei diesem Prozeß zwangsweise anfallen. Es ist ferner bekannt, daß Ethylen mit guten Ausbeuten in der Gasphase mit molekularem Sauerstoff an einem silberhaltigen Katalysator epoxidert werden kann. Wegen seiner mangelnden Selektivität ist jedoch dieses Verfahren für andere Olefine ungeeignet.

Weiterhin lassen sich Olefine durch Umsetzung mit Hydroperoxiden, die aus Kohlenwassersotffen wie z.B. Isobutan oder Ethylbenzol, durch Oxidation mit Luft erhältlich sind (US-PS 3 351 635), in Gegenwart eines Katalysators, der Vanadin-, Molybdän- oder Wolframverbindungen enhält, in entsprechende Epoxide überführen. Diese Methode hat den gewichtigen Nachteil, daß neben der erforderlichen Abtrennung des Katalysatorsystems der aus dem Hydroperoxid als Koppelprodukt in äquimolarem Maßstab anfallende Alkohol, sofern er nicht wirtschaftlich verwertbar ist, nur unter erheblichem technischen Aufwand in das Hydroperoxid rückgeführt werden kann.

Durch Anwendung der "Prileschajew-Reaktion" (N.Prileschajew, Ber. dtsch. chem. Ges. 42, 4811 (1909)) können die zuvor erwähnten Nachteile teilweise vermieden bzw. vermindert werden.

Diese Reaktion beinhaltet im wesentlichen die Umsetzung eines Olefins mit einer organischen Percarbonsäure. Allerdings führt auch hierbei z.B. die Verwendung von Perameisensäure, die darüberhinaus in höheren Konzentrationen detonationsfähige Gemische ausbildet, zu erheblichen Abwassermengen, die einer gewissenhaften Entsorgung bedürfen. Auch ein Einsatz von Peressigsäure in wäßrigem Medium führt zu großen Mengen verdünnter Essigsäure, die auf wirtschaftliche Weise nicht aufkonzentriert und rückgeführt werden können. Falls die Peressigsäure, wie häufig notwendig wegen der Produktstabilität, während des Prozesses mit Alkalicarbonatlösung abgepuffert und/oder nach der Reaktion mit Alkalihydroxidlösung neutralisiert wird, fallen stark salzhaltige, die Umwelt belastende Abwässer an.

Vorteile sollten daher eine Verwendung organischer Percarbonsäuren in einem organischen Lösungsmittel bieten. So ist z.B. bekannt, vorstehend beschriebenes Diepoxid durch Umsetzung des ihm zugrundeliegenden Diolefins mit Acetaldehydmonoperacetat bzw. Peressigsäure in Ethylacetat oder Aceton als Solvens darzustellen (US-PS 2 716 123, US-PS 2 804 473). Eine solche Betriebsweise erscheint zunächst sehr attraktiv, stellt jedoch trotzdem keine optimale Möglichkeit wegen der als Koppelprodukt anfallenden Essigsäure dar, da diese unter erheblichem Aufwand aus dem Reaktionsgemisch abgetrennt und gereinigt werden muß und daher auf unwirtschaftliche Weise gewonnen wird.

Auch sind die Ausbeuten gemäß diesem Verfahren mit 85,5 % und einer Produktreinheit von 86 % äußerst unbefriedigend (US-PS 2 716 123, Beispiel IV). Darüberhinaus ist der Prozeß der Acetaldehydoxidation nicht ungefährlich, da nach diesem Verfahren explosive Zwischenprodukte auftreten.

Im allgemeinen vertritt die Fachwelt über Epoxidation mit Persäuren die Meinung, daß die derartig mit Persäuren erhaltenen Reaktionsgemische aufgrund ihres Gehalts an Wasser und Säure z.B. Essigsäure, sehr leicht mit den gebildeten Epoxiden unter Bildung von Nebenprodukten wie Glykolen, Glykolmono- und -diestern reagieren, siehe DE-AS 15 43 032. Epoxidationsverfahren, die z.B. Perameisen- bis Perpropionsäure verwendeten, galten daher als besonders schwierig durchführbar in saurem Milieu, da hierdurch Aufspaltung des Oxiranringes eintrat, siehe DE-PS 29 16 834.

In diesem Zusammenhang ist es auch zu verstehen, daß in der DE-OS 31 01 037 und EP-OS 056 932, die die Herstellung von n-Alkyloxiranen mittels Perpropionsäure beschreiben, im Anspruch 1 eine Percarbonsäurelösung mit einem Mineralsäuregehalt kleiner als 50 ppm beansprucht wird. Laut Beschreibung im Text soll der Mineralsäuregehalt bevorzugt sogar kleiner als 10 ppm betragen. Diese Verfahren bezogen sich auf die Herstellung von Monoepoxiden.

Nach dem genannten Stand der Technik war aber zu erwarten, daß sich Diepoxide noch schlechter mit Percarbonsäure herstellen lassen würden, da diese wegen der zwei vorhandenen Epoxid-Teilstrukturen besonders leicht zu Folgereaktionen neigen.

Aufgabe der Erfindung ist es, vorgenanntes Diepoxid mit Hilfe von Perpropionsäure in guten Ausbeuten und unter Vermeiden von störender Nebenproduktbildung herzustellen.

Es wurde nun gefunden, daß sich diese Aufgabe lösen läßt, wenn man das Diolefin der Formel

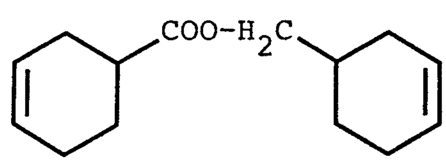

(II)

mit einer Lösung von Perpropionsäure in Benzol im Molverhältnis 1 : 2 bis 1 : 3 (Diolefin zu Perpropionsäure) bei einer Temperatur von 10 bis 100 °C umsetzt.

Perpropionsäure kann z.B. gemäß dem in DE-PS 25 19 289 beschriebenen Verfahren hergestellt werden, indem man wäßriges Wasserstoffperoxid mit Propionsäure in Gegenwart von Schwefelsäure umsetzt und anschließend die entstandene Perpropionsäure mit Benzol aus dem Reaktionsgemisch extrahiert. Die auf diese Weise erhaltene Perpropionsäure in benzolischer Lösung kann noch weiter gereinigt werden, um den Restgehalt an Schwefelsäure, Wasser und Wasserstoffperoxid zu verringern, siehe z.B. DE-PS 25 19 290. Bevorzugt ist aber eine Perpropionsäurelösung, die keiner weiteren Reinigung bedarf; mit anderen Worten, der Rohextrakt aus der Perpropionsäureherstellung kann als solcher direkt eingesetzt werden. Dies führt zu einem erheblich verringerten technischen Aufwand.

Es kann daher eine Perpropionsäurelösung in Benzol verwendet werden, die bis 1,5 Gewichtsprozent Wasserstoffperoxid, 1,5 Gewichtsprozent Wasser und bis zu 800 ppm Mineralsäure enthält.

Nach dem erfindungsgemäßen Verfahren wird das Diolefin vorzugsweise als solches, oder aber auch verdünnt in einem geeigneten Lösungsmittel, z.B. Benzol, eingesetzt, wobei man die Konzentration in einem weiten Bereich frei wählt.

Die Lösungen der Perpropionsäure, die u.a. noch aus Propionsäure bestehen, können zwischen 10 und 30 Gewichtsprozent der Persäure enthalten. Vorzugsweise werden Lösungen mit einem Persäuregehalt von ca. 20 Gewichtsprozent eingesetzt. Ein bevorzugtes Molverhältnis von Diolefin zu Perpropionsäure liegt bei 1 : 2 bis 1 : 2,4. Besonders bevorzugt ist ein Persäureüberschuß von 3 bis 15 Molprozent.

Die Umsetzung findet bevorzugt bei Temperaturen von 20 bis 50 °C statt. Das erfindungsgemäße Verfahren läßt sich unter verschiedenen Drücken durchführen; im allgemeinen wird unter Normaldruck gearbeitet, das Verfahren läßt sich aber auch bei Über- oder Unterdruck durchführen.

Die Umsetzung kann sowohl diskontinuierlich oder kontinuierlich in für diese Art der Reaktion geeigneten Reaktoren, wie Rührkesseln, Rührkesselkaskaden, Rühr- oder Schlaufenreaktoren erfolgen, wobei die Reaktionswärme auf beliebige Weise, z.B. Siedekühlung oder innen- bzw. außenliegende Kühleinrichtungen, abgeführt wird.

Geeignete Werkstoffe für die Reaktionsapparate zur Durchführung des erfindungsgemäßen Verfahrens sind z.B. Glas, Edelstahl oder emailliertes Material.

Die Perpropionsäure wird mit dem Diolefin auf beliebige Art zusammengebracht. So kann man beide Reaktionsteilnehmer zusammen oder nacheinander in beliebiger Reihenfolge in den Reaktor einbringen. Bei diskontinuierlicher Arbeitsweise wird vorzugsweise das Diolefin vorgelegt und die Persäure unter Kontrolle der Reaktionstemperatur zudosiert; es kann aber ebensogut umgekehrt verfahren werden, d.h. man legt die Persäure vor und dosiert das Olefin temperaturkontrolliert zu. Erfolgt die Umsetzung kontinuierlich, so können beide Reaktanten getrennt oder gemeinsam dem Reaktor zugeführt werden. Bei Verwendung mehrerer hintereinandergeschalteter Reaktoren, wie z.B. einer Rührkesselkaskade oder einer Folge von Rührkesseln mit einem Rohrreaktor als Nachreaktor, kann man sowohl Persäure- als auch Diolefinzugabe auf mehrere Reaktoren verteilen. Zum Lösen von Diolefin können neben dem bevorzugten Benzol auch Toluol, Chlorbenzol oder halogenierte Aliphaten eingesetzt werden.

Nach dem erfindungsgemäßen Verfahren ist eine kontinuierliche Arbeitsweise besonders vorteilhaft. Gemäß dieser wird das cycloaliphatische Diolefin mit einer Lösung von Perpropionsäure in Benzol im Molverhältnis 1 : 2 bis 1 : 3 bei den genannten Temperaturen von 10 bis 100 °C in ein Reaktionssystem eingespeist, das aus einer Folge von 1 bis 4 ideal durchmischten Reaktoren und einem Nachreaktor besteht, wobei man die Verweilzeit so einstellt, daß der Umsatz, bezogen auf eingesetzte olefinische Doppelbindungen nach dem oder den ideal durchmischten Reaktor(en) mindestens 80 Molprozent und nach dem Nachreaktor mindestens 95, bevorzugt über 98 Molprozent, beträgt. Anschließend wird das den Nachreaktor verlassende Reaktionsgemisch in einer Kombination von Destillations- und Desorptionsschritten von Benzol, Propionsäure, unumgesetzter Perpropionsäure sowie sonstigen flüchtigen Bestandteilen befreit. Diese Auftrennung des Reaktionsgemisches kann, da das gebildete Diepoxid die Komponente mit dem höchsten Siedepunkt des Gemisches darstellt, nach einer der folgenden Varianten durchgeführt werden.

Variante 1 (diskontinuierlich)

Gemäß dieser werden die einzelnen Bestandteile des Reaktionsgemisches in der Reihenfolge ihrer Siedepunkte einzeln oder als Gemische destillativ oder destillativ und desorptiv entfernt. Hierbei gehen Fraktionen von Benzol, Resten Perpropionsäure, Propionsäure und sonstige leicht flüchtige Bestandteile über. Als Sumpf verbleibt das Diepoxid. Das abgetrennte Benzol sowie die Propionsäure können gegebenenfalls nach weiteren Reinigungsschritten in die Persäureherstellung rückgeführt werden.

Variante 2 (kontinuierlich, Abbildung 1)

Nach dieser kontinuierlich durchzuführenden Variante werden zunächst, nachdem das Reaktionsgemisch die Reaktionseinheit 1 verlassen hat, Benzol, Propionsäure und nicht umgesetzte Perpropionsäure größtenteils in der ein- oder mehrstufigen Destillationseinheit 2 entfernt. Diese besteht aus geeigneten Apparaten wie Dünnschicht-, Fallfilm- oder Umlaufverdampfern. Es ist vorteilhaft, unter vermindertem Druck von 0,5 bis 600, vorzugsweise 10 bis 300 mbar, zu destillieren (Temperatur des Heizmediums 50 bis 150 °C). Die mittleren Verweilzeiten, bezogen auf die einzelnen Stufen der Verdampfung, liegen bei maximal 10 Minuten, bevorzugt werden Verweilzeiten von maximal 5 Minuten. Anschließend wird nach dem erfindungsgemäßen Verfahren die im Rohprodukt verbliebene Menge Propionsäure in der Desorptionseinheit 3 mit Benzoldampf, der im Verdampfer 4 generiert wird, desorptiv entfernt. Die Brüden aus der Desorptionseinheit 3 können entweder an der Destillationseinheit 2 vorbeigeführt oder durch diese hindurchgeführt werden. Nach diesem Schritt werden aus dem Diepoxid die verbliebenen Spuren Benzol mit Wasserdampf aus dem Verdampfer 6 in der Desorptionseinheit 5 und/oder Stickstoff bzw. sonstigen Inertgasen in der Desorptionseinheit 8 desorbiert. Es ist besonders bevorzugt, zunächst mit Wasserdampf und anschließend mit Inertgasen zu desorbieren. Das aus der Desorptionseinheit 5 stammende Kondensat trennt sich im Phasentrenner 7 in eine organische Phase und Wasser, welches in den Verdampfer 6, gegebenenfalls nach Ergänzung, zurückgeführt wird. Die organische Phase, die vorwiegend Benzol und Propionsäure enthält, wird gegebenenfalls nach weiterer Aufarbeitung, der Perpropionsäureherstellung oder Epoxidation zugeführt. Ebenfalls werden die aus den Destillations- bzw. Desorptionseinheiten 2 und 3 stammenden Kondensatströme, die im wesentlichen aus Benzol, nicht umgesetzter Perpropion- und Propionsäure bestehen, nach weiterer Auftrennung, - siehe Abbildung 3 - deren Beschreibung später erfolgt, in die Persäureherstellung bzw. Epoxidation rückgeführt.

Als Desorptionseinheit in allen Beispielen eignen sich Apparate wie z.B. Fallfilmverdampfer, Sambay-Verdampfer, Kolonnen mit Einbauten oder Füllkörpern oder ähnliche Vorrichtungen, die einen guten Stoffaustausch zwischen gasförmiger und flüssiger Phase ermöglichen und die dem Fachmann bekannt sind.

Variante 3 (kontinuierlich, Abbildung 2)

Gemäß der kontinuierlich anzuwendenden Variante 3 werden wie bei "Variante 2" Benzol, unumgesetzte Perpropionsäure und Propionsäure in der ein- oder mehrstufigen Destillationseinheit 2 entfernt. Anschließend wird in der Desorptionseinheit 3 restliche Propionsäure mit Benzoldampf desorbiert. Zur Entfernung von verbliebenen Spuren an Propionsäure wird nun das Rohepoxid mit wäßrigen Alkalien in der Extraktion 9 und anschließend mit Wasser in der ein- oder mehrstufigen Extraktionseinheit 10 gewaschen. Geeignete Apparate für diese Schritte sind Extraktionskolonnen verschiedener Bauart oder auch Mixer-Settler-Einheiten, deren Betriebsweise und Auslegung dem Fachmann wohl bekannt sind. Als wäßrige Alkalilösungen können Lösungen von z.B. NaOH, KOH, $Na_2CO_3$, $K_2CO_3$, $NaHCO_3$, $KHCO_3$, $NH_3$ usw. eingesetzt werden, wobei deren Konzentration in einem weiten Bereich frei wählbar ist. Bevorzugt wird eine NaOH-Lösung mit einer Konzentration zwischen 0,2 bis 15 Gewichtsprozent, besonders bevorzugt 0,5 bis 1,0 Gewichtsprozent. Bei Einsatz von Mixer-Settler-Einheiten für die Wäsche mit Wasser kann das Wasser im Gegenstrom geführt werden, es kann aber auch jede Einheit mit Frischwasser betrieben werden. Vorteilhaft wird ein Teil des Abwassers aus den Mixer-Settler-Einheiten zum Ansetzen der Alkali-Lösung verwendet. Die Alkali- und Wasserwäsche kann in einem Temperaturbereich von 10 bis 90 $^{o}C$ betrieben werden, bevorzugt werden Temperaturen von 30 bis 70 $^{o}C$. In der Alkali-Wäsche beträgt das Gewichtsverhältnis von durchgesetztem Epoxid zu Alkalilösung 1 : 1 bis

100 : 1, in der Wasserwäsche liegt das Verhältnis von Epoxiddurchsatz zu Wasserdurchsatz bei 1 : 1 bis 100 : 1.

Im Anschluß an die Wasserwäsche erfolgt die weitere Aufarbeitung durch Desorption mit Wasserdampf und/oder Inertgas wie bei Variante 2 beschrieben.

Bei allen Varianten durch Kombination von Destillations- und Desorptionsschritten fallen Kondensate an, die überwiegend aus Benzol, unumgesetzter Perpropionsäure und sonstigen Leichtsiedern bestehen; sie werden nach dem erfindungsgemäßen Verfahren in eine aus einer oder mehreren Kolonnen bestehende Destillationseinheit 11 (Zeichnung 3) überführt. Diese liefert als Kopfprodukt Benzol und gegebenenfalls weitere Leichtsieder. Ersteres wird gegebenenfalls nach weiterer Destillation in 12 in das Herstellungsverfahren der Perpropionsäure zurückgeführt. Im Sumpf der Destillationseinheit 11 fällt ein Gemisch aus Propionsäure, Perpropionsäure und Benzol mit einem Benzolanteil von 5 bis 35 Gewichtsprozent, bezogen auf die Sumpfmischung, an. Dieses Gemisch wird einer weiteren Destillationseinheit 13 zugeführt, in der man die Gesamtmenge des zugeführten Benzols und der Perpropionsäure mit Anteilen Propionsäure über Kopf abzieht und dabei eine Konzentration von Perpropionsäure im Destillat von 25 Gewichtsprozent nicht überschreitet und dieses Kopfprodukt in das Herstellungsverfahren der Perpropionsäure oder in die Umsetzung des Diolefins mit Perpropionsäure zurückführt. Als Sumpfprodukt in Kolonne 13 fällt Propionsäure an, die nach weiterer Aufarbeitung wie Reindestillation in die Herstellung der Perpropionsäure, gegebenenfalls nach Ergänzung, zurückgeführt wird. Besonders vorteilhaft ist es, die in 13 anfallende Propionsäure dampfförmig oberhalb des Sumpfes abzuziehen und zu kondensieren, da hierdurch der weitere Reinigungsschritt entfällt.

Erfindungsgemäß werden alle destillativen Aufarbeitungsschritte vorzugsweise unter vermindertem Druck, z.B. 0,5 bis 600 mbar, durchgeführt. Kolonnen, in denen Benzol oder Propionsäure als Kopfprodukt anfällt, können ebenso bei Normaldruck betrieben werden.

Das erfindungsgemäße Verfahren bietet eine Reihe von überraschenden Vorteilen.
Mit Hilfe der sogenannten Prileschajew-Reaktion ist es nach diesem Verfahren möglich, das vorgenannte Diepoxid im technischen Maßstab auf gefahrlose Weise in hoher Ausbeute herzustellen. Das auf diese Weise erhaltene Produkt zeichnet sich durch außerordentliche Reinheit, hohen Epoxidgehalt, niedrige Viskosität, Geruchslosigkeit und helle Farbe aus.

Ebenfalls besonders niedrig, bzw. nicht nachweisbar, ist der Gehalt an Monoepoxid sowie ionischen Verunreinigungen, ($Na^{+}$, $Cl^{-}$, $Fe^{3+}$, etc.) wodurch ein Produkt mit deutlich besseren Eigenschaften als nach anderen Verfahren hergestelltes Diepoxid vorstehender Struktur zur Verfügung steht. Gerade für Anwendungen im Bereich der Mikroelektronik werden an die Reinheit der dort einzusetzenden Diepoxide Qualitätsanforderungen gestellt, wie sie nach dem erfindungsgemäßen Verfahren besonders leicht erfüllt werden.

Das beschriebene Verfahren ist wirtschaftlich, da alle Hilfsmedien rückgeführt werden. Das Verfahren ist besonders umweltfreundlich, da aus dem Oxidationsmittel lediglich Wasser als Abfall entsteht; darüberhinaus fallen nur geringe Mengen an sonstigen Abwässern, Leichtsiedern und Destillationsrückständen an, die unproblematisch und gefahrlos entsorgt werden können.

Erfindungsgemäß sind nur kurze Reaktionszeiten nötig, was die technische Durchführung besonders wirtschaftlich gestaltet.

Es war überraschend und nicht vorhersehbar, daß die Umsetzung des vorgenannten Diolefins mit einer rohen Perpropionsäure, die noch Mineralsäure, Wasser und Wasserstoffperoxid in den vorgenannten Konzentrationen enthält, bei weitestgehender Unterdrückung von Neben- und Folgereaktionen durchführbar ist. Weiterhin war nicht vorhersehbar, daß die dabei anfallenden Reaktionsgemische erfindungsgemäß destillativ oder destillativ und desorptiv aufarbeitbar, ohne daß sich dadurch der Epoxidgehalt des Produktes merklich verringert.

Beispiel 1 (diskontinuierlich):

Eine Mischung aus 270 g Propionsäure, 248 g Wasserstoffperoxid (50 %ig) und 155 g konzentrierter Schwefelsäure wurde 15 Minuten bei 35 °C gerührt und anschließend mit 500 g Benzol extrahiert. Der Extrakt (748 g) wies einen Gehalt an Perpropionsäure von 20,7 Gewichtsprozent auf. Zu dieser Lösung dosierte man innerhalb 15 Minuten unter Rühren und Kühlung auf 40 °C 170 g Tetrahydrobenzoesäuretetrahydrobenzylester ein, was einem Verhältnis Persäure zu Diolefin von 2,23 : 1 entspricht und ließ nach der Zugabe 2 Stunden bei 40 °C nachreagieren. Der Olefinumsatz betrug über 99 %, der Persäureumsatz 95,8 %.

Die erhaltene klare Lösung (905 g) wurde innerhalb 2 Stunden bei 95 °C und 120 mbar über einen Dünnschichtverdampfer gegeben, wobei gleichzeitig im Gegenstrom 220 g pro Stunde Benzoldampf geführt wurden. Im Sumpf fielen 211 g Rohdiepoxid an. Dieses wurde erneut bei 95 °C und 65 mbar über einen Dünnschichtverdampfer gegeben; gleichzeitig wurde im Gegenstrom mit Wasserdampf desorbiert.

Als Sumpf erhielt man 194 g nahezu farbloses Diepoxid mit einem Epoxidgehalt von 7,1 val/kg und einer Viskosität von 330 mPa.s (25 °C).

Beispiel 2 (kontinuierlich):

In den ersten Rührkessel einer Reaktionseinheit, bestehend aus zwei Rührkesseln vom Volumen 300 ml und 600 ml sowie einem als Rohrreaktor ausgebildetem Nachreaktor, der ein Volumen von 790 ml hat, wurden stündlich 3,22 Mol Perpropionsäure in Benzol (ca. 22 %ig , die gemäß DE-PS 25 19 289 hergestellt wurde und 0,57 Gewichtsprozent $H_2O_2$; 0,91 Gewichtsprozent $H_2O$ und 500 ppm Schwefelsäure enthielt und für die Beispiele 2 bis 5 benutzt wurde) und 1,43 Mol Tetrahydrobenzoesäuretetrahydrobenzylester eingespeist, was einem Molverhältnis Persäure zu Diolefin von 2,25 : 1 entspricht. Die Reaktionstemperatur betrug im 1. Reaktor 41 °C, im 2. Reaktor 40 °C und im Nachreaktor 46 °C. Die Umsätze an Olefin betrugen nach der Rührkesselkaskade 91,1 %, nach dem Rohrreaktor 99,2 %. Gemäß Aufarbeitungsvariante 2 wurden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wurde in einem zweiten Verdampfer gleichen Typs und gleicher Fläche bei 90 °C und 100 mbar bei einem Durchsatz von 315 g pro Stunde Benzoldampf desorbiert. Die Brüden des 2. Verdampfers wurden vollständig im Gegenstrom zum Produktstrom in den 1. Verdampfer geführt.

Nachfolgend wurde das Epoxid in zwei Desorptionseinheiten, bestehend aus jeweils einem Sambay-Verdampfer (Fläche 0,065 $m^2$) mit einer am Sumpfabzug angesetzten Füllkörperkolonne (NW 25, Länge 90 cm, Raschigringe) bei 100 mbar mit 49 g pro Stunde Wasserdampf und bei 20 mbar mit 36 g pro Stunde Stickstoff bei Temperaturen von 90 °C behandelt.

Als Produkt vielen stündlich 331,4 Diepoxid mit folgenden Kennzahlen an:

Viskosität (mPa.s, 25 °C): 270
Epoxid-Gehalt (val/kg) 7,57
Farbzahl (Hazen): 40

Beispiel 3 (kontinuierlich):

In den ersten Rührkessel einer Reaktionseinheit, bestehend aus zwei Rührkesseln vom Volumen 200 ml und 400 ml sowie einem als Rohrreaktor ausgebildetem Nachreaktor, der ein Volumen von 1900 ml hat, wurden stündlich 3,13 Mol Perpropionsäure in Benzol (ca. 22 %ig) und 1,40 Mol Tetrahydrobenzoesäuretetrahydrobenzylester eingespeist, was einem Molverhältnis Persäure zu Diolefin von 2,24 : 1 entspricht. Die Reaktionstemperatur betrug im 1. Reaktor 41 °C, im 2. Reaktor 37 °C und im Nachreaktor 47 °C. Die Umsätze an Olefin betrugen nach der Rührkesselkaskade 82,8 %, nach dem Rohrreaktor 99,2 %. Gemäß Aufarbeitungsvariante 2 wurden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wurde in einem 2. Verdampfer gleichen Typs und gleicher Fläche bei 90 °C und 100 mbar bei einem Durchsatz von 315 g pro Stunde Benzoldampf desorbiert. Die Brüden wurden wie bei Beispiel 2 geführt.

Nachfolgend wurde das Epoxid in zwei Desorptionseinheiten, bestehend aus jeweils einem Sambay-Verdampfer (Fläche 0,065 $m^2$) mit einer am Sumpfabzug angesetzten Füllkörperkolonne (NW 25, Länge 90 cm, Raschigringe) bei 100 mbar mit 49 g pro Stunde Wasserdampf und bei 20 mbar mit 36 g pro Stunde Stickstoff bei Temperaturen von 90 °C behandelt.

Als Produkt fielen stündlich 349,9 g Diepoxid mit folgenden Kennzahlen an:

Viskosität (mPa.s, 25 °C): 295
Epoxid-Gehalt (val/kg): 7,59
Farbzahl (Hazen): 25

Beispiel 4 (kontinuierlich):

In den 1. Rührkessel einer Reaktionseinheit, bestehend aus zwei Rührkesseln vom Volumen 300 ml und 600 ml sowie einem als Rohrreaktor ausgebildetem Nachreaktor, der ein Volumen von 1900 ml hat, wurden stündlich 3,04 Mol Perpropionsäure in Benzol (ca. 22 %ig) und 1,40 Mol Tetrahydrobenzoesäuretetrahydrobenzylester eingespeist, was einem Molverhältnis Persäure zu Diolefin von 2,1 : 1 entspricht. Die Reaktionstemperatur betrug im 1. Reaktor 40 °C, im 2. Reaktor 40 °C und im Nachreaktor 37 °C. Die Umsätze an Olefin betrugen nach der Rührkesselkaskade 84,9 %, nach dem Rohrreaktor 97,8 %. Gemäß Aufarbeitungsvariante 3 wurden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wurde in einem 2. Verdampfer gleichen Typs und gleicher Fläche bei 90 °C und 100 mbar bei einem Durchsatz von 315 g pro Stunde Benzoldampf desorbiert. Die Brüden aus dem 2. Verdampfer wurden nicht durch den 1. Verdampfer geführt. Das so als Sumpf erhaltene Rohepoxid wurde nun in einem Mixer-Settler-System mit 1 %iger Natronlauge (180 ml pro Stunde) und anschließend in einer Folge von drei Mixer-Settler-Einheiten mit Wasser (jeweils 180 ml pro Stunde) gewaschen. Nachfolgend wurde das Epoxid in zwei Desorptionseinheiten, bestehend aus jeweils einem Sambay-Verdampfer (Fläche 0,065 $m^2$) mit einer am Sumpfabzug angesetzten Füllkörperkolonne (NW 25, Länge 90 cm, Raschigringe) bei 100 mbar mit 49 g pro Stunde Wasserdampf und bei 20 mbar mit 36 g pro Stunde Stickstoff bei Temperaturen von 90 °C behandelt.

Als Produkt fielen stündlich 337,4 g Diepoxid mit folgenden Kennzahlen an:

| | |
|---|---|
| Viskosität (mPa.s, 25 °C): | 288 |
| Epoxid-Gehalt (val/kg): | 7.32 |
| Farbzahl (Hazen): | 30 |

<u>Beispiel 5 (kontinuierlich):</u>

In den 1. Rührkessel einer Reaktionseinheit, bestehend aus zwei Rührkesseln vom Volumen 1500 ml und 700 ml sowie einem als Rohrreaktor ausgebildetem Nachreaktor, der ein Volumen von 1650 ml hat, wurden stündliche 3,19 Mol Perpropionsäure in Benzol (ca. 22 %ig) und 1,45 Mol Tetrahydrobenzoesäuretetrahydrobenzylester eingespeist, was einem Molverhältnis Persäure zu Diolefin von 2,20 : 1 entspricht. Die Reaktionstemperatur betrug im 1.Reaktor 39 °C, im 2. Reaktor 34 °C und im Nachreaktor 40 °C. Die Umsätze an Olefin betrugen nach der Rührkesselkaskade 91,0 %, nach dem Rohrreaktor 99,6 %. Gemäß <u>Aufarbeitungsvariante 3</u> wurden zunächst in einem Sambay-Verdampfer mit der Fläche von 0,065 $m^2$ bei einer Temperatur von 90 °C und einem Druck von 100 mbar Benzol, Perpropionsäure und Propionsäure abgetrennt. Restliche Propionsäure wurde in einem 2. Verdampfer gleichen Typs und gleicher Fläche bei 90 °C und 100 mbar bei einem Durchsatz von 315 g pro Stunde Benzoldampf desorbiert. Die Brüden aus dem 2. Verdampfer wurden nicht durch den 1.Verdampfer geführt. Das so als Sumpf erhaltene Rohepoxid wurde nun in einem Mixer-Settler-System mit 1 %iger Natronlauge (180 ml pro Stunde) und anschließend in einer Folge von drei Mixer-Settler-Einheiten mit Wasser (jeweils 180 ml pro Stunde) gewaschen.

Nachfolgend wurde das Epoxid in zwei Desorptionseinheiten, bestehend aus jeweils einem Sambay-Verdampfer (Fläche 0,065 $m^2$) mit einer am Sumpfabzug angesetzten Füllkörperkolonne (NW 25, Länge 90 cm, Raschigringe) bei 100 mbar mit 49 g pro Stunde Wasserdampf und bei 20 mbar mit 36 g pro Stunde Stickstoff bei Temperaturen von 90 °C behandelt.

Als Produkt fielen stündlich 349,2 g Diepoxid mit folgenden Kennzahlen an:

| | |
|---|---|
| Viskosität (mPa.s, 25 °C): | 408 |
| Epoxid-Gehalt (val/kg): | 7,31 |
| Farbzahl (Hazen): | 30 |

Auch wenn nicht näher bezeichnet, sind alle Prozentangaben Gewichtsprozente.

Degussa Aktiengesellschaft
Weissfrauenstrasse 9, 6000 Frankfurt

# Verfahren zur Herstellung eines cycloaliphatischen Diepoxids

## Patentansprüche:

1. Verfahren zur Herstellung eines cycloaliphatischen Diepoxids der Formel

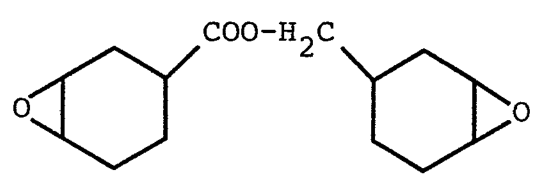

(I)

durch Epoxydierung des Diolefins der Formel

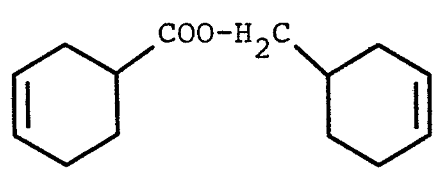

(II)

mit einer Percarbonsäure in organischer Lösung, <u>dadurch gekennzeichnet</u>, daß man das Diolefin der obengenannten Formel (II) mit einer Lösung von Perpropionsäure in Benzol im Molverhältnis 1 : 2 bis 1 : 3 (Diolefin zu Perpropionsäure) bei einer Temperatur von 10 bis 100 °C, vorzugsweise bei 20 bis 50 °C, umsetzt.

2. Verfahren nach Anspruch 1, <u>dadurch gekennzeichnet</u>, daß die Perpropionsäurelösung maximal einen Gehalt von 1,5 Gewichtsprozent Wasserstoffperoxid, 1,5 Gewichtsprozent Wasser und ca. 800 ppm Mineralsäure besitzt.

3. Kontinuierliches Verfahren nach den Ansprüchen 1 und 2, <u>dadurch gekennzeichnet</u>, daß man das cycloaliphatische Diolefin mit einer Lösung von Perpropionsäure in Benzol im Molverhältnis 1 : 2 bis 1 : 3 in ein Reaktionssystem einspeist, das aus einer Folge von 1 bis 4 ideal durchmischten Reaktoren und einem Nachreaktor besteht, die Reaktion bei einer Temperatur von 10 bis 100 °C durchführt, wobei man die Verweilzeit so einstellt, daß der Umsatz, bezogen auf eingesetzte olefinische Doppelbindungen, nach dem oder den ideal durchmischten Reaktoren bei mindestens 80 Molprozent und nach dem Nachreaktor bei mindestens 95, bevorzugt > 98 Molprozent, liegt, und daß man das aus dem Nachreaktor austretende Gemisch in einer Kombination von Destillations- und Desorptionsschritten von Benzol, Propionsäure, geringen Mengen an Perpropionsäure und von anderen Leichtsiedern befreit.

4. Verfahren nach den Ansprüchen 1 bis 3, <u>dadurch gekennzeichnet</u>, daß man die Destillations- und Desorptionsschritte unter vermindertem Druck von 0,5 bis 600 mbar bei Temperaturen des Heizmediums von 50 bis 150 °C und bei Verweilzeiten von maximal 10 Minuten, bevorzugt maximal 5 Minuten, in den einzelnen Schritten, durchführt.

5. Verfahren nach den Ansprüchen 1 bis 4, <u>dadurch gekennzeichnet</u>, daß man die Destillations- und Desorptionsschritte bei 10 bis 300 mbar durchführt.

6. Verfahren nach den Ansprüchen 1 bis 5, <u>dadurch gekennzeichnet</u>, daß man zunächst Benzol und Propionsäure sowie die geringen Mengen Perpropionsäure größtenteils destillativ abtrennt, worauf man die verbliebene Menge Propionsäure im Rohepoxid mit Benzoldampf weiter desorptiv entfernt und entweder hieran direkt anschließend das Benzol und Spuren von Propionsäure desorptiv mit Wasserdampf und/oder Inertgasen austreibt, oder daß man nach der Desorption mit Benzoldampf das rohe Epoxid zunächst mit wäßrigen Alkalien und anschließend mit Wasser wäscht und erst dann die Desorption mit Wasserdampf und/oder Inertgasen anschließt.

7. Verfahren nach den Ansprüchen 1 bis 6, <u>dadurch gekennzeichnet</u>, daß man das aus der Kombination von Destillations- und Desorptionsschritten erhaltene Gemisch aus Benzol, Propionsäure, geringen Mengen Perpropionsäure, sowie gegebenenfalls anderen Leichtsiedern in eine aus zwei oder mehreren Destillationskolonnen bestehende Destillationsanlage führt und in dem ersten Destillationsschritt über Kopf Benzol, gegebenenfalls im Gemisch mit anderen Leichtsiedern, abzieht, das man gegebenenfalls nach destillativer Reinigung in das Herstellungsverfahren der Perpropionsäure wieder zurückführt, und daß man im Sumpf die Gesamtmenge an Perpropionsäure und Propionsäure, sowie Anteile von Benzol in Mengen von 5 bis 35 Gewichtsprozent, bezogen auf die Sumpfmischung, entnimmt und diese Mischung in eine zweite Destillationsstufe führt, in der man die Gesamtmenge des darin enthaltenen Benzols und der Perpropionsäure mit Anteilen an Propionsäure über Kopf abzieht und dabei eine Konzentration von Perpropionsäure in dem Kopfprodukt von mehr als 25 Gewichtsprozent nicht überschreitet, dieses Kopfprodukt in das Herstellungsverfahren der Perpropionsäure oder in die Umsetzung der Perpropionsäure mit Olefin zurückführt, und

daß man die Propionsäure als Sumpfprodukt, gegebenenfalls dampfförmig, oberhalb des Sumpfes abzieht und in das Herstellungsverfahren der Perpropionsäure zurückführt.

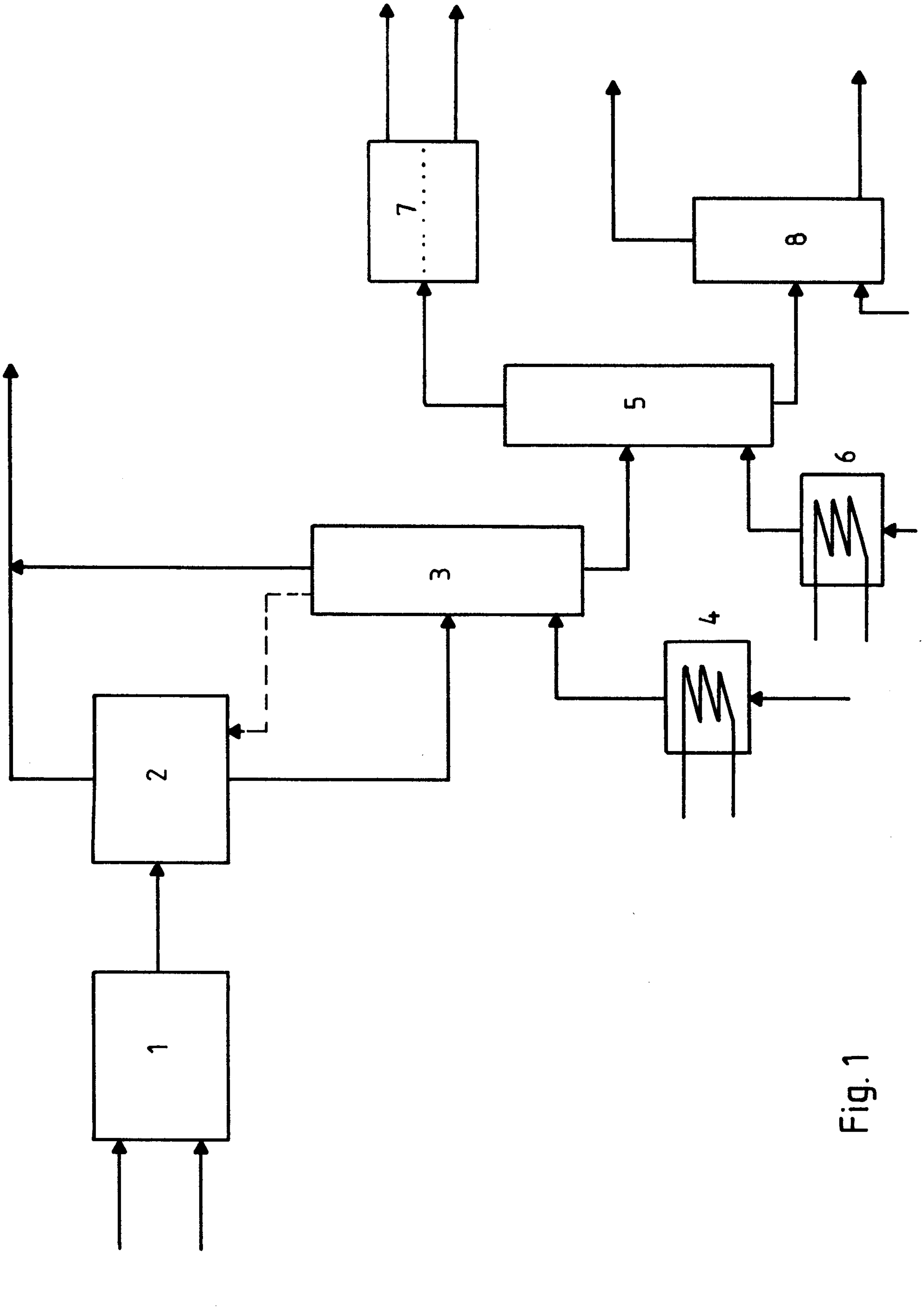


Fig. 1

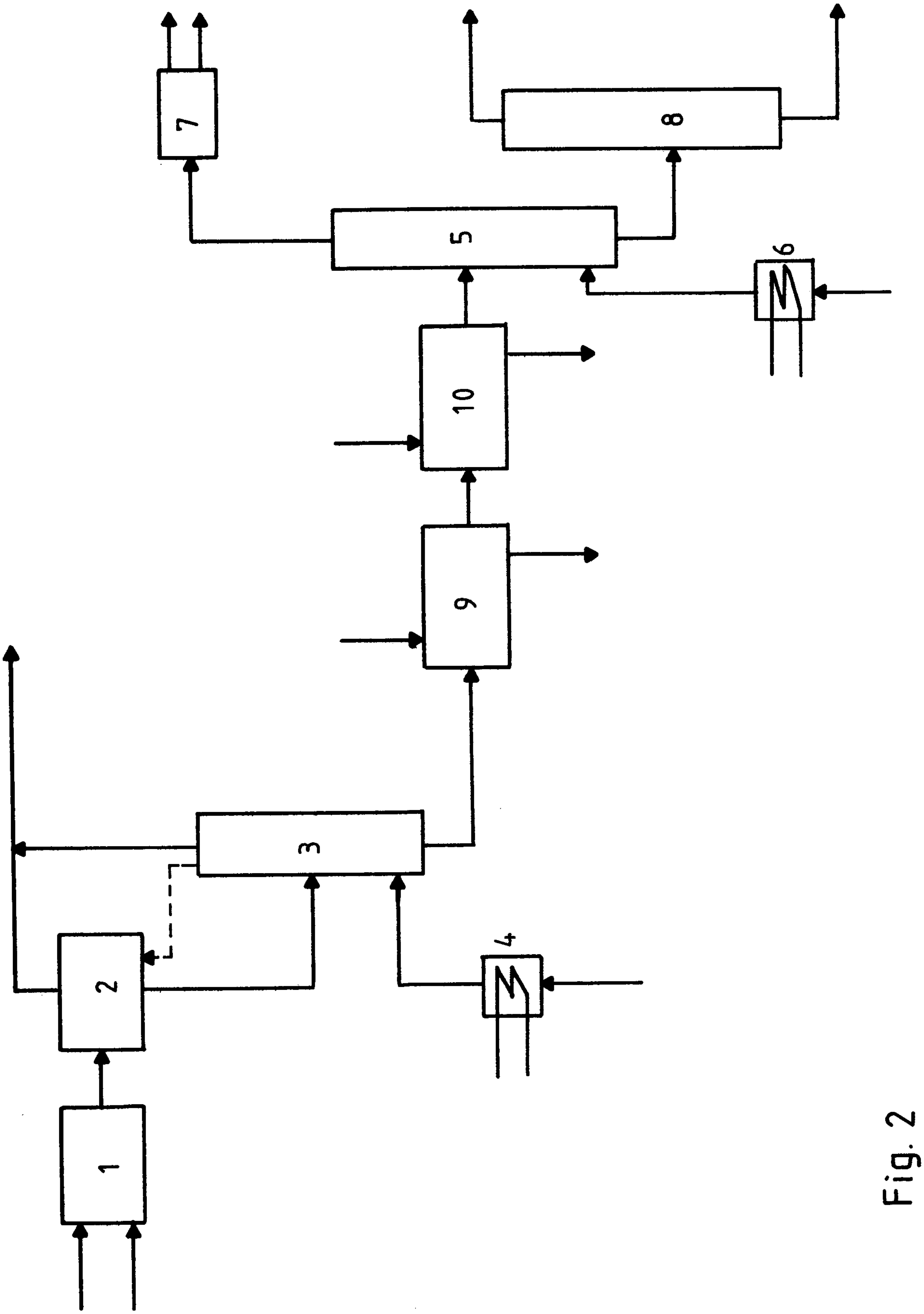


Fig. 2

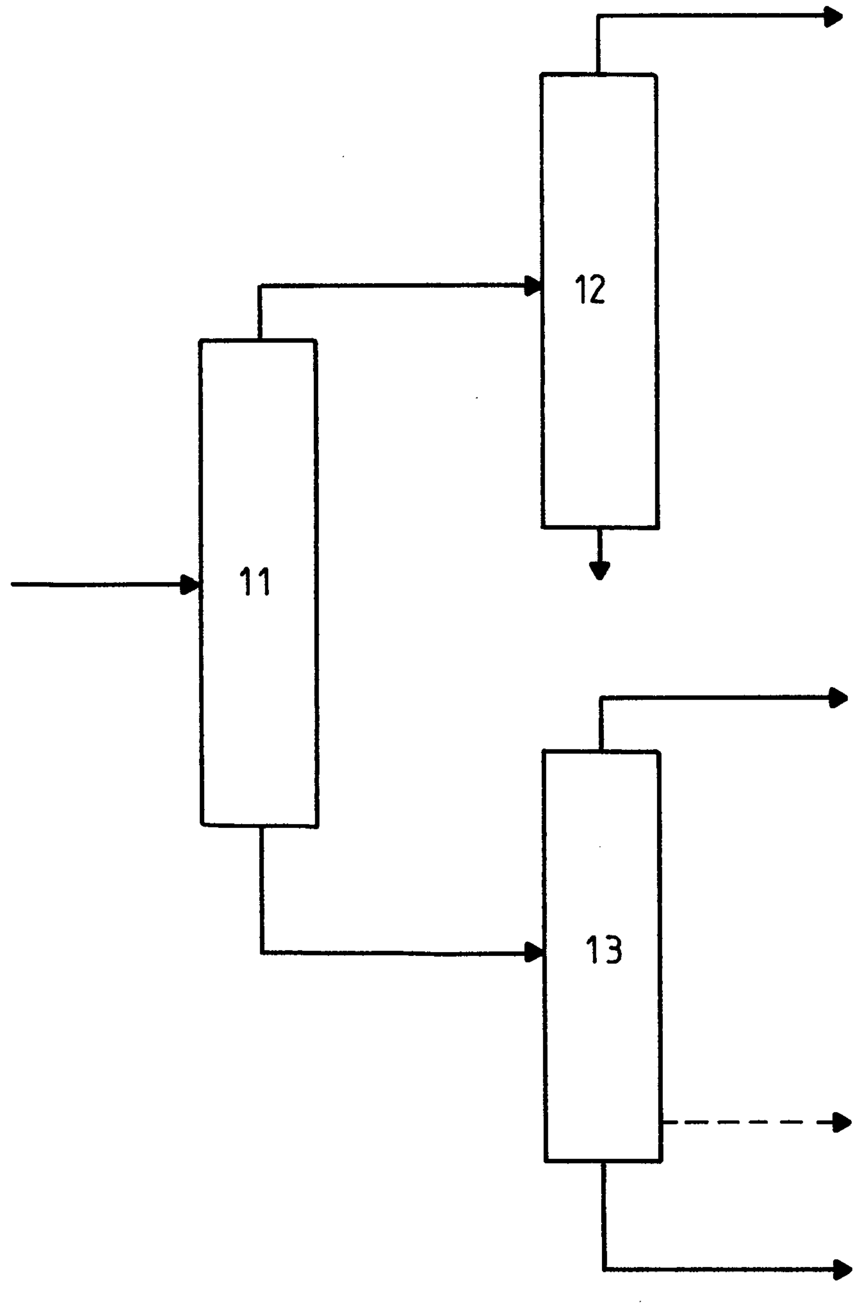


Fig. 3